# EUROPÄISCHE PATENTSCHRIFT

(11) **EP 3 644 874 B1**
(45) Veröffentlichungstag und Bekanntmachung des Hinweises auf die Patenterteilung: **24.06.2026**
(21) Anmeldenummer: 18740740.8
(22) Anmeldetag: 27.06.2018
(51) Int. Cl.: A61B 17/28

(54) **MEDIZINISCHES HANDINSTRUMENT MIT REINIGUNGSOPTIMIERTEM FEDERELEMENT**
MEDICAL HAND-HELD INSTRUMENT COMPRISING A CLEANING-OPTIMISED SPRING ELEMENT
INSTRUMENT À MAIN MÉDICAL MUNI D'UN ÉLÉMENT ÉLASTIQUE AYANT UN NETTOYAGE OPTIMISÉ

(30) Priorität: 27.06.2017 DE 102017114260
(43) Veröffentlichungstag der Anmeldung: 06.05.2020
(73) Patentinhaber: Aesculap AG, 78532 Tuttlingen (DE)
(72) Erfinder: VOGTHERR, Robert, 78532 Tuttlingen (DE)
(74) Vertreter: Winter, Brandl - Partnerschaft mbB
(86) Internationale Anmeldenummer: PCT/EP2018/067204
(87) Internationale Veröffentlichungsnummer: WO 2019/002347

(56) Entgegenhaltungen:
- DE-A1- 102011 056 235
- DE-A1- 102014 100 603
- DE-A1- 102014 110 881
- DE-U1- 202009 001 809
- DE-U1- 202010 007 995
- US-A- 3 921 478
- US-A- 5 470 328
- US-A- 5 782 749
- US-A1- 2003 075 026

## Beschreibung

### Technisches Gebiet

Die vorliegende Erfindung bezieht sich auf ein medizinisches Handinstrument gemäß dem Oberbegriff des Anspruchs 1, d.h. auf ein chirurgisches Handinstrument der Zangen- oder Scherenbauart, mit zwei Griffelementen oder Griffbügeln, welche relativ zueinander schwenkbar sind, und einem Federelement, vorzugsweise eine Blattfeder die U- oder V-förmig gebogen ist, welches zwei Federelementenden aufweist, die jeweils mit einem der beiden Griffelemente verbunden sind, so dass bei Verschwenken zumindest eines der beiden Griffelemente aus einer Grundstellung ein Zurückschwenken in die Grundstellung mittels des Federelements bewerkstelligbar ist.

### Stand der Technik

In der Druckschrift DE 20 2010 007 995 U1 wird ein Instrument offenbart, bei welchem eine einteilige Blattfeder auf einer Seite des Instruments bzw. Instrumentengriffes verschraubt ist und mit ihrem freien Ende auf der gegenüberliegenden Seite des Instruments bzw. am anderen Instrumentengriff anliegt. Dies birgt etliche Nachteile: Generell sollte bei chirurgischen Instrumenten so gut als möglich auf Gewinde verzichtet werden, da die Spalte zwischen Mutterngewinde und Schraube quasi nicht reinigbar sind. Bei dem Instrument gemäß Druckschrift DE 20 2010 007 995 U1 liegt die Blattfeder im Bereich der Verschraubung direkt an der Innenseite des Instruments an, wodurch sich auch hier ein sehr enger, nicht reinigbarer Spalt zwischen Blattfeder und Instrument ergibt. Feuchtigkeit oder Reinigungsflüssigkeit kann aus diesem Spalt sehr schlecht austrocknen und kann über einen gewissen Zeitraum korrosiv wirken. Zudem ist die Blattfeder in diesem korrosionsanfälligen Bereich durchbohrt und in ihrer Geometrie geschwächt, wodurch die Blattfeder an dieser Stelle bruchgefährdet ist. Im Auflagebereich auf der gegenüberliegenden Instrumentenseite reibt das freie Blattfeder-Ende während der Bewegung bzw. Benutzung des Instruments. Diese Reibung verschlechtert auf Dauer die Oberflächengüte des Auflagebereichs und vergrößert dort ebenfalls die Korrosionsanfälligkeit.

In der Druckschrift DE 20 2009 002 433 U1 ist ein Instrument mit zwei Griffen offenbart, bei welchem pro Griff jeweils ein angeschraubtes Blattfederteil vorgesehen ist. Um ein Zurückschwenken in eine Grundstellung zu bewirken, stützen sich die beiden Blattfederteile aneinander ab. Hinsichtlich seiner Reinigbarkeit weist das Instrument gemäß Druckschrift DE 20 2009 002 433 U1 insbesondere wegen seinen Verschraubungen die gleichen Nachteile wie das Instrument gemäß Druckschrift DE 20 2010 007 995 U1 auf. Weiterhin besteht im Kontaktbereich der beiden freien Blattfederenden eine Steckverbindung, die relativ scharfkantig ist. Da sich diese Stelle mitten im gut zugänglichen Griffbereich des Instruments befindet, kann ein OP-Handschuh des Anwenders hier leicht eingeschnitten oder beschädigt werden. Und auch wenn diese Verbindungsstelle im Neuzustand des Instruments gut entgratet ist, wird sich nach etlichen Anwendungen ein gewisser Abrieb und Grat bilden.

Die Druckschrift DE 20 2009 001 809 U1 bezieht sich auf ein Instrument, dessen Federmechanismus wie bei dem Instrument gemäß Druckschrift DE 20 2009 002 433 U1 aus zwei verschraubten Blattfederteilen besteht. Um zumindest die genannten Nachteile einer scharfkantigen Steck-Verbindungsstelle zu vermeiden, ist zwischen den Blattfederteilen eine Kugel- und Pfannengeometrie vorgesehen. Aufgrund der Verschraubungen und der Kugel- und Pfannengeometrie ist das Instrument gemäß DE 20 2009 001 809 U1 hinsichtlich seiner Reinigbarkeit aber weiterhin nachteilhaft. Darüber hinaus ist der Federmechanismus gemäß DE 20 2009 001 809 U1 aufwändig herzustellen, da die dargestellten Geometrien an den freien Blattfederenden angeschweißt oder komplex gefräst werden müssen.

Die Druckschrift DE 20 2011 052 256 U1 beschreibt ein Instrument, dessen Federmechanismus aus einem verschraubten Blattfederteil und einem an dessen freien Ende gelenkig gelagert angehängten zweiten Teil besteht. Aufgrund der Verschraubung und der Komplexität des Federmechanismus ist auch das Instrument gemäß Druckschrift DE 20 2011 052 256 U1 verhältnismäßig schlecht reinigbar und aufwendig herzustellen.

Instrumente, bei welchen die Federmechanismen in aufwendiger Art zumindest teilweise integral mit entsprechenden Griffteilen ausgebildet sind, werden beispielsweise in den Druckschriften DE 101 37 915 B4, DE 10 2007 030 874 B4 oder DE 10 2014 102 606 A1 beschrieben. Diese Instrumente sind nicht nur aufgrund ihrer aufwendigen Herstellung sondern auch aufgrund der schlechten Ersetz- bzw. Demontierbarkeit der Blattfederteile nachteilig.

Die DE 10 2014 100 603 A1 offenbart ein Rohrschaftinstrument, deren Griffelemente nicht mittels eines Federelements miteinander verbunden sind.

Die DE 10 2014 110 881 A1 offenbart ein vergleichbares Rohrschaftinstrument.

### Offenbarung der Erfindung

In Anbetracht der Instrumente gemäß dem genannten Stand der Technik ist es Aufgabe der vorliegenden Erfindung, ein gut reinigbares, sich verhältnismäßig schwach abnutzendes und/oder relativ einfach zu reparierendes und herzustellendes medizinisches Handinstrument bereitzustellen.

Diese Aufgabe wird durch ein medizinisches Handinstrument mit den Merkmalen des Anspruchs 1 gelöst. Vorteilhafte Weiterbildungen sind Gegenstand der Unteransprüche.

Die vorliegende Erfindung betrifft folglich ein medizinisches Handinstrument mit zwei relativ zueinander schwenkbaren Griffelementen (Griffbügeln, Griffhebeln, Hebelarmen) und einem Federelement (Blattfeder). Das vorzugsweise U- oder V-förmig gebogene Federelement weist zwei Enden auf, welche nachfolgend Federelementenden genannt werden. Jedes der beiden Federelementenden ist jeweils mit einem entsprechenden der beiden Griffelemente derart verbunden, dass bei Verschwenken zumindest eines der beiden Griffelemente aus einer Grundstellung relativ zu dem anderen Griffelement ein Zurückschwenken in die Grundstellung mittels des Federelements bewerkstelligbar ist.

Das heißt, wenn eines der beiden Griffelemente von einem Anwender des Instruments manuell verschwenkt wird, kann das Federelement das verschwenkte Griffelement in seine Grundstellung zurückschwenken, sobald der Anwender das verschwenkte Griffteil loslässt. Bei dem medizinischen Handinstrument handelt es sich um ein zangenartiges oder scherenartiges Instrument. Das Handinstrument weist also gegenüber einem Scharnier, welches die Griffelemente schwenkbar verbindet, Maulelemente bzw. Lastarme auf. Bei dem zangenartigen oder scherenartigen Instrument, kann sich die Grundstellung auf eine Offenstellung oder eine Schließstellung der Maulelemente beziehen. Das medizinische Handinstrument kann so ausgebildet sein, dass bei der Anwendung des Handinstruments beide Griffelemente verschwenkt werden. Das medizinische Handinstrument kann aber auch so ausgebildet sein, dass bei der Anwendung nur ein Griffelement verschwenkt wird. Ist nur ein Griffelement dafür vorgesehen, verschwenkt zu werden, dient das andere Griffelement beim Verschwenken nur als Gegenlager für die Hand des Anwenders.

Das erfindungsgemäße medizinische Handinstrument zeichnet sich dadurch aus, dass zumindest eines der beiden Federelementenden mit dem entsprechenden Griffelement über einen durch plastisches Umformen des Federelements erzeugten Hinterschnitt formschlüssig verbunden ist und der Hinterschnitt in allen Relativpositionen des Federelements gegenüber dem entsprechenden Griffelement mit diesem Griffelement derart im Eingriff steht, dass eine Demontage ausgeschlossen ist.

Die Formulierung "alle Relativpositionen des Federelements gegenüber dem entsprechenden Griffelement" bezieht sich hierbei auf alle Positionen des Federelements, die das mit dem entsprechenden Griffelement verbundene Federelement relativ zum entsprechenden Griffelement einnehmen kann, ohne dass dabei die durch das plastische Umformen erzeugte formschlüssige Verbindung zwischen dem Federelement und dem entsprechenden Griffelement zerstört wird.

Anders ausgedrückt ist bzw. wird zumindest eines der beiden Federelementenden so ausgebildet, dass es sich an dem entsprechenden Griffelement einhaken kann bzw. mit dem entsprechenden Griffelement verrasten kann. "Einhaken" bzw. "Verhaken" bzw. "Verrasten" heißt in diesem Zusammenhang, dass die Verbindung zwischen dem Federelement und dem entsprechenden Griffelement zumindest in einer Richtung durch Formschluss gewährleistet ist.

Erfindungsgemäß wird dieser Formschluss allerdings nicht mittels Hilfsfügeteile wie Nieten sondern unmittelbar durch Umformen des Federelements bewerkstelligt.

Das Umformen kann vor dem, beim oder nach dem Anbringen des Federelements an dem entsprechenden Griffteil durchgeführt werden. Genauer gesagt, kann das separate Federelement also an zumindest einem Federelementende zunächst zu einem Haken bzw. zu einer Verraststruktur plastisch verformt werden und dann mit dem entsprechenden Griffelement verhakt bzw. verrastet werden. Auch ist es möglich, ein noch unverformtes Federelementende an dem entsprechenden Griffelement anzubringen bzw. anzulegen, um dieses dann an dem Griffelement lose angebrachte bzw. angelegte Federelementende durch Umformen des Federelementendes mit dem Griffelement zu verhaken bzw. zu verrasten bzw. zu verbinden. Schließlich ist es auch möglich, dass ein im Vorfeld schon auf jegliche mögliche Art und Weise hakenförmig bzw. verrastelementförmig ausgebildetes Federelementende zum Verbinden mit dem entsprechenden Griffelement in eine am Griffelement entsprechend ausgebildete Struktur eingefädelt wird. Das Federelement ist dann so ausgebildet, dass es beim Einfädeln zumindest zeitweise elastisch umgeformt wird.

Wird zum Verbinden des Federelements und des entsprechenden Griffelements das Federelement umgeformt, kann auf vorteilhafte Weise auf Hilfsfügeteile verzichtet werden. Dieses Weglassen von Teilen kann nicht nur bei der Herstellung sondern kann aufgrund der möglicherweise aus der reduzierten Bauteilanzahl resultierenden verkleinerten Oberfläche auch bei der Reinigung des erfindungsgemäßen Handinstruments vorteilhaft sein.

Die Reinigbarkeit und die Herstellbarkeit des Handinstruments kann weitergehend erleichtert werden, indem gemäß einem zusätzlichen Aspekt der Erfindung das Federelement einstückig ausgebildet wird. Insbesondere kann das Federelement als U- oder V-förmige, also zwei Schenkel aufweisende; Blattfeder ausgebildet sein. Die zwei Schenkel der Blattfeder können sich in die gleiche Richtung hin aufweiten wie die beiden Griffelemente.

Um die Handhabung des medizinischen Handinstruments zu verbessern, kann es gemäß einem zusätzlichen Aspekt der Erfindung vorteilhaft sein, das zumindest eine mit dem entsprechenden Griffelement verhakte Federelementende und/oder das zumindest eine mit dem entsprechenden Federelementende verhakte bzw. verrastete Griffelement derart strukturell auszubilden, dass das Federelement bezüglich des Griffelements nur in einer Schwenkebene verschwenkbar ist. Anders ausgedrückt kann es vorteilhaft sein, die Verhakung bzw. Verrastung des Federelements mit dem entsprechenden Griffelement derart auszubilden, dass zumindest in der Grundstellung der Griffelemente nur eine relative Schwenkbewegung des Federelements bezüglich des Griffelements, nämlich die Schwenkbewegung des Federelements in der Schwenkebene der Griffelemente oder in einer zur Schwenkebene der Griffelemente parallelen Ebene, zugelassen ist. Schwenkbewegungen des Federelements in alle anderen Richtungen werden dann durch Material des Griffelements formschlüssig und/oder reibschlüssig verhindert.

Ist das zumindest eine mit dem entsprechenden Griffelement verhakte bzw. verrastete Federelementende durch plastisches Umformen des Federelements mit dem Griffelement verbunden, kann gemäß einem zusätzlichen Aspekt der Erfindung eine verhältnismäßig stabile Verbindung des Federelementendes mit dem Griffelement gewährleistet werden.

Gemäß einem zusätzlichen Aspekt der Erfindung kann an dem zumindest einen mit dem entsprechenden Federelementende verhakten Griffelement eine Öse vorgesehen sein, mit welcher das Federelementende verhakt ist. Die Öse kann in vorteilhafter Weise das hakenförmige bzw. verrastete Federelementende seitlich so einfassen, dass ein unbeabsichtigtes Lösen der Verbindung zwischen dem hakenförmigen bzw. verrasteten Federelementende und dem entsprechenden Griffelement weniger wahrscheinlich ist.

Gemäß einem zusätzlichen Aspekt kann die Öse durch Verbinden zweier Enden eines gabelförmigen Teils des entsprechenden Griffelements mittels eines Stifts gebildet sein. Auf diese Weise kann die Herstellung des Handinstruments vereinfacht werden.

Die Herstellung kann auch dadurch vereinfacht werden, dass nur ein Federelementende hakenförmig bzw. verrastelementförmig ausgebildet wird und die Verbindung des anderen Federelementendes mit dem entsprechenden Griffelement einfacher ausgebildet wird. Konkret heißt das, dass man das nicht hakenförmige bzw. das nicht zur Verrastung vorgesehene Federelementende lediglich mittels einer Steckverbindung mit dem entsprechenden Griffelement verbinden kann. Bei der Steckverbindung kann das Federelementende in eine an dem Griffelement ausgebildeten Federelementendenaufnahme hineinragen und durch Verkanten in der Federelementendenaufnahme gehalten sein. "Verkanten" bedeutet in diesem Zusammenhang, dass die Federkraft des Federelements das entsprechende Federelementende in der Federelementaufnahme derart verdreht, dass das Federelementende lokal, das heißt an bestimmten Stellen, an Innenwände der Federelementaufnahme gedrückt wird und an diesen Stellen ein Reibschluss zwischen dem Federelementende und der Federelementaufnahme verursacht wird. Alternativ oder zusätzlich kann auch eine Verbindung des nicht hakenförmig ausgebildeten Federelementendes mit dem entsprechenden Griffelement mittels Presspassung bewerkstelligt sein.

Gemäß einem zusätzlichen Aspekt kann das zumindest eine mit dem entsprechenden Griffelement verhakte bzw. verrastete Federelementende durch elastisches Umformen des Federelements mit dem Griffelement verbunden sein. Auf diese Weise kann die Verbindung zwischen dem hakenförmigen bzw. verrasteten Federlemenetende und dem entsprechenden Griffelement werkzeuglos montierbar und demontierbar ausgebildet sein, womit sowohl die Herstellung als auch die Reinigung des Handinstruments vereinfacht werden kann.

Gemäß einem zusätzlichen Aspekt kann das zumindest eine mit dem entsprechenden Griffelement verhakte bzw. verrastete Federelementende zwei Vorsprünge aufweisen. Um das Federelement mit dem entsprechenden Griffelement zu verbinden, können an dem Griffelement Lageraugen vorgesehen sein, mit welchen die zwei Vorsprünge zusammenwirken können. Die zwei Vorsprünge an dem Federelementende werden nachfolgend Federelementendenvorsprünge genannt. Indem zwei Federelementendenvorsprünge vorgesehen werden, kann ein vollständiges Versagen der Verbindung zwischen dem hakenförmigen bzw. verrasteten Federelementende und dem entsprechenden Griffelement leichter verhindert werden. Wird ein Federelementendenvorsprung derart beschädigt, dass er nicht mehr zur Verbindung zwischen dem Federelement und dem Griffelement beiträgt, kann der andere Federelementendenvorsprung zumindest eine notdürftige Verbindung aufrechterhalten.

Gemäß einem zusätzlichen Aspekt können die zwei Lageraugen zum Einführen des entsprechenden Federelementendenvorsprungs jeweils einen in Lageraugen-Achsrichtung verlaufenden Schlitz aufweisen. Die Schlitze können insbesondere als Parallelspalte so dimensioniert sein, dass die insbesondere flach ausgebildeten Federelementendenvorsprünge nur dann durch die Schlitze in die Lageraugen eingeführt werden können, wenn das mit den Federelementendenvorsprüngen ausgestattete Federelementende im Wesentlichen parallel zu den Schlitzen ausgerichtet wird. Mittels der Schlitze kann die Montierbarkeit bzw. die Demontierbarkeit des medizinischen Handinstruments weiter vereinfacht werden. Sind die Schlitze in beschriebener Art als Parallelspalte ausgebildet, kann dabei gleichzeitig die Sicherheit der Verbindung gewährleistet werden.

Die vorliegende Erfindung betrifft auch ein Verfahren zum Herstellen eines medizinischen Handinstruments. Das Verfahren weist folgende Schritte auf:
- Verbinden zweier Griffelemente (Griffbügel, Griffhebel, Hebelarme), so dass diese relativ zueinander schwenkbar sind,
- Ausbilden eines Federelements (insbesondere einer, vorzugweise U- oder V-förmigen Blattfeder), so dass dieses zwei Federelementenden aufweist,
- plastisches und/oder elastisches Umformen des Federelements und
- Verbinden eines jeden Federelementendes mit dem entsprechenden Griffelement, so dass bei Verschwenken zumindest eines der beiden Griffelemente aus einer Grundstellung ein Zurückschwenken in die Grundstellung mittels des Federelements bewerkstelligbar ist.

Das Verfahren zeichnet sich dadurch aus, dass das Umformen des Federelements ein formschlüssiges Verbinden zumindest eines der beiden Federelementenden mit dem entsprechenden Griffelement bedingt. Zumindest ein Federelementende wird erfindungsgemäß also dadurch mit dem entsprechenden Griffelement formschlüssig verbunden, dass das Federelement plastisch und/oder elastisch umgeformt wird.

Das formschlüssige Verbinden findet erfindungsgemäß dabei gleichzeitig mit dem Umformen des Federelements statt.

Insbesondere können gleichzeitig mit dem Umformen des Federelements beide Federelementenden mit den jeweiligen Griffelementen formschlüssig verbunden werden.

### Kurzbeschreibung der Zeichnungen

Die vorliegende Erfindung wird im Folgenden anhand bevorzugter Ausführungsbeispiele unter Bezugnahme auf die beigefügten Zeichnungen näher beschrieben. Es zeigen:
Figur 1 eine perspektivische Detailansicht eines medizinischen Handinstruments gemäß einer ersten Ausführungsform;
Figur 2 eine Querschnittsansicht einer Verhakung eines Federelementendes mit einem Griffelement gemäß der ersten Ausführungsform;
Figur 3A eine perspektivische Ansicht einer Verhakung eines Federelementendes mit einem Griffelement gemäß einer zweiten Ausführungsform;
Figur 3B eine Querschnittsansicht der in Figur 3A gezeigten Verhakung;
Figur 4A eine perspektivische Ansicht einer Verhakung eines Federelementendes mit einem Griffelement gemäß einer dritten Ausführungsform;
Figur 4B eine Querschnittsansicht der in Figur 4A gezeigten Verhakung;
Figur 5A eine perspektivische Ansicht einer Verhakung eines Federelementendes mit einem Griffelement gemäß einer vierten Ausführungsform;
Figur 5B eine Querschnittsansicht der in Figur 5A gezeigten Verhakung;
Figur 5C eine perspektivische Ansicht eines in ein Griffelement eingestecktes Federelementendes gemäß der vierten Ausführungsform;
Figur 5D eine Querschnittsansicht des in Figur 5c gezeigten Federelementendes;
Figur 6A eine perspektivische Ansicht einer Verhakung eines Federelementendes mit einem Griffelement gemäß einer fünften Ausführungsform;
Figur 6B eine Draufsicht des Federelementendes gemäß der fünften Ausführungsform; und
Figur 6C eine Querschnittsansicht der in Figur 6A gezeigten Verhakung.

Gleiche oder funktional äquivalente Merkmale sind in den einzelnen Figuren mit denselben Bezugszeichen versehen. Zweistellige Bezugsnummern beziehen sich dabei auf die erste Ausführungsform. Dreistellige Bezugsnummern beginnend mit der Ziffer "1" beziehen sich auf die zweite Ausführungsform. Dreistellige Bezugsnummern beginnend mit der Ziffer "2" beziehen sich auf die dritte Ausführungsform. Dreistellige Bezugsnummern beginnend mit Ziffer "3" beziehen sich auf die vierte Ausführungsform. Dreistellige Bezugsnummern beginnend mit Ziffer "4" beziehen sich auf die fünfte Ausführungsform.

### Detaillierte Beschreibung bevorzugter Ausführungsformen

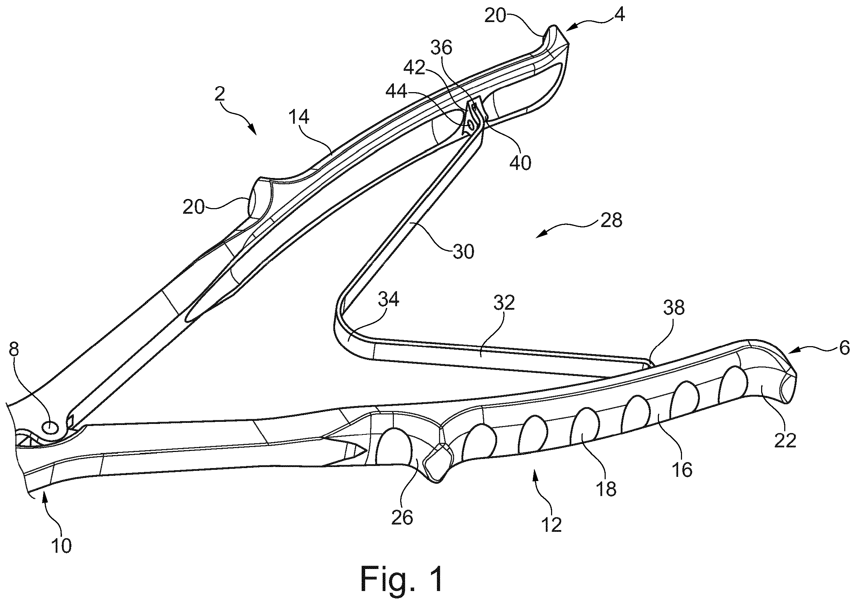

Figur 1 zeigt ein medizinisches Handinstrument 2 gemäß einer ersten Ausführungsform. Das Handinstrument 2 ist nach Zangen- oder Scherenbauart ausgebildet. Das heißt, es weist zwei Hebel 4 und 6 auf, welche miteinander über ein Scharnier 8 schwenkbar verbunden sind. Der Abschnitt des Handinstruments 2 einerseits des Scharniers 8 ist ein (nur ansatzweise gezeigter) Maulabschnitt 10. Der Abschnitt andererseits des Scharniers 8 ist ein Griffabschnitt 12. Die proximalen Teile der Hebel 4 und 6, welche den Griffabschnitt 12 des Handinstruments 2 bilden, werden nachfolgend Griffelemente 14 und 16 genannt.

Das Griffelement 14 des Hebels 4 und das Griffelement 16 des Hebels 6 sind im Wesentlichen symmetrisch zueinander ausgebildet. Beide Griffelemente 14 und 16 weisen jeweils an der Seite, welche vom jeweils anderen Griffelement 16 und 14 weg weist, Vertiefungen 18 auf, um eine gute Haptik beim Greifen des Handinstruments 2 zu gewährleisten. An der proximalen Seite des Griffabschnitts 12, an den freien Enden der Griffelemente 14 und 16, weisen die Griffelemente 14 und 16 an der Seite, welche vom jeweils anderen Griffelement 16 und 14 weg weist, Griffvorsprünge 20 und 22 auf. Auch jeweils eine Handbreit entfernt von den freien Enden weisen die Griffelemente 14 und 16 an der Seite, welche vom jeweils anderen Griffelement 16 und 14 weg weist, Griffvorsprünge 24 und 26 auf. Die Griffvorsprünge 20 und 24 beziehungsweise 22 und 26 grenzen jeweils einen Bereich an den Griffelementen 14 und 16 ein, an welchen ein Anwender den Griffabschnitt 12 bevorzugt ergreift bzw. bevorzugt ergreifen soll. Die Griffvorsprünge 20 und 24 beziehungsweise 22 und 26 sind dabei dafür bestimmt, ein Abrutschen der Finger des Anwenders zu verhindern.

Zwischen den Griffelementen 4 und 6 ist ein Federelement 28 vorgesehen. Das Federelement 28 ist im Wesentlichen U- bzw. V-förmig und weist zwei Schenkel 30 und 32 auf, welche miteinander über einen bogenförmigen Abschnitt 34 verbunden sind. An ihrem jeweiligen freien Ende sind die Schenkel 30 bzw. 32 in Richtung weg vom jeweils anderen Schenkel 32 bzw. 30 abgeknickt. Diese abgeknickten Bereiche werden nachfolgend Verbindungsabschnitte 36 und 38 genannt.

Die beiden Schenkel 30 und 32 sowie der bogenförmige Abschnitt 34 und die Verbindungsabschnitte 36 und 38 sind einstückig in Form einer gebogenen Blattfeder bevorzugt aus Federstahl ausgebildet.

Die Griffelemente 14 und 16 weisen jeweils an der Seite, welche zum jeweils anderen Griffelement 16 bzw. 14 weist, etwa zwei Fingerbreiten vom freien Ende des Griffelements 14 bzw. 16 entfernt, zwei sich parallel zueinander und parallel zur Schwenkachse des Scharniers 8 erstreckende Verbindungsplatten 40 und 42 auf. Die beiden Verbindungsplatten 40 und 42 eines Griffelements 14 bzw. 16 weisen mittig jeweils eine Aussparung 44 auf. Die beiden Verbindungsplatten 40 und 42 eines jeweiligen Griffelements 14 bzw. 16 sind jeweils so weit voneinander entfernt bzw. der Spalt zwischen den beiden Verbindungsplatten 40 und 42 eines jeweiligen Griffelements 14 bzw. 16 ist so dimensioniert, dass bei der Montage des Federelements 28 an den Griffelementen 14 und 16 die Verbindungsabschnitte 36 und 38 jeweils mit Übergangspassung zwischen die entsprechenden Verbindungsplatten 40 und 42 eingesteckt werden können.

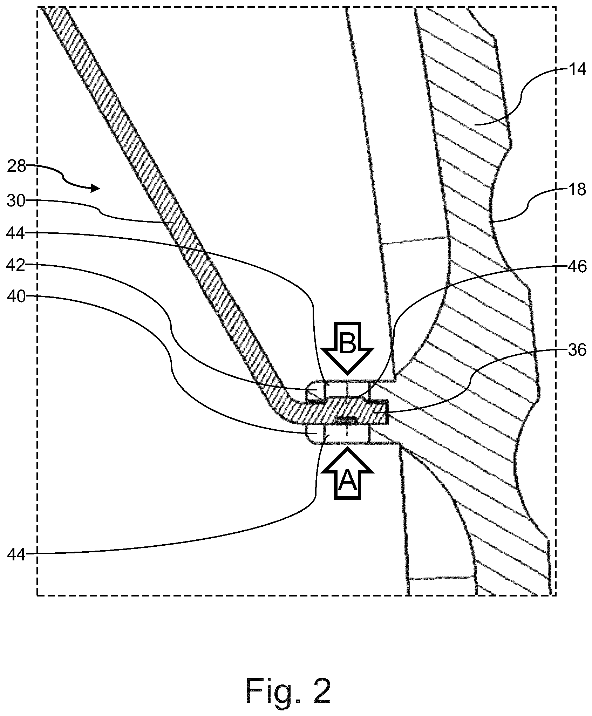

Um den Verbindungsabschnitt 36 mit dem Griffelement 14 bzw. um den Verbindungsabschnitt 38 mit dem Griffelement 16 zu verhaken, wird der Verbindungsabschnitt 36 bzw. 38, wie in Figur 2 gezeigt, über die Aussparungen 44 geclincht. Genauer gesagt wird zunächst der Verbindungsabschnitt 36 bzw. 38 zwischen die Verbindungsplatten 40 und 42 eingesteckt. Anschließend wird ein (nicht gezeigtes) Werkzeug durch die Aussparung 44 der Verbindungsplatte 40 durchgeführt und gegen den Verbindungsabschnitt 36 bzw. 38 gedrückt (siehe Pfeil A in Figur 2), so dass der Verbindungsabschnitt 36 bzw. 38 lokal verformt wird und der durch die Verformung hervorgerufene Clinchvorsprung 46 in die Aussparung 44 der Verbindungsplatte 42 hineinragt. Die Verhakung bzw. die Verrastung des Federelements 28 und dem Griffelement 14 bzw. 16 wird also primär durch Einhaken bzw. Verrasten des Clinchvorsprungs 46 mit der Verbindungsplatte 42 bewerkstelligt. Anders ausgedrückt bildet der Clinchvorsprung 46 einen erfindungsgemäßen Hinterschnitt, der mit dem Griffelement 14 über dessen Verbindungsplatten 40 und 42 formschlüssig verbunden ist.

Um die Verhakung bzw. Verrastung des Federelements 28 mit dem Griffelement 14 bzw. 16 zu lösen, muss das (nicht gezeigte) Werkzeug lediglich durch die Aussparung 44 der Verbindungsplatte 42 durchgeführt und gegen den Clinchvorsprung 46 gedrückt (siehe Pfeil B in Figur 2) werden, so dass der Verbindungsabschnitt 36 bzw. 38 lokal zurück verformt wird, wodurch der Clinchvorsprung 46 eingeebnet wird und nicht mehr in die Aussparung 44 der Verbindungsplatte 42 hineinragt. Das Lösen der Verhakung bzw. Verrastung des Federelements 28 mit dem Griffelement 14 bzw. 16 kann demnach nur durch Zerstörung des Clinchvorsprungs 46 bewerkstelligt werden. Ein zerstörungsfreies Lösen ist nicht möglich.

Die relative Stellung der Griffelemente 14 und 16, in welcher das mit den Griffelementen 14 und 16 verhakte bzw. verrastete Federelement 28 nicht gespannt ist, wird nachfolgend Grundstellung genannt. In der Grundstellung fluchtet die Verbindungsplatte 40 des Griffelements 14 mit der Verbindungsplatte 40 des Griffelements 16. Entsprechend fluchtet die Verbindungsplatte 42 des Griffelements 14 mit der Verbindungsplatte 42 des Griffelements 16.

Dadurch, dass der Spalt zwischen den Verbindungsplatten 40 und 42 des Griffelementes 14 bzw. 16 auch in Richtung senkrecht zur Schwenkebene der Griffelemente 14 und 16 freigestellt ist, kann das Federelement 28 zur Montage auch seitlich, das heißt in Richtung senkrecht zur Schwenkebene der Griffelemente 14 und 16, mit seinen Verbindungsabschnitten 36 und 38 zwischen die Verbindungsplatten 40 und 42 der Griffelemente 14 und 16 eingeschoben werden.

Bei der oben beschriebenen ersten Ausführungsform sind die Aussparungen 44 derart ausgebildet und wird der jeweilige Verbindungsabschnitt 36 bzw. 38 so weit zwischen die entsprechenden Verbindungsplatten 40 und 42 eingeschoben, dass nach dem Umformen des Verbindungsabschnitts 36 bzw. 38 rundum den Clinchvorsprung 46 weiterhin Bereiche des Verbindungsabschnitts 36 bzw. 38 zwischen den entsprechenden Verbindungsplatten 40 und 42 gefangen sind.

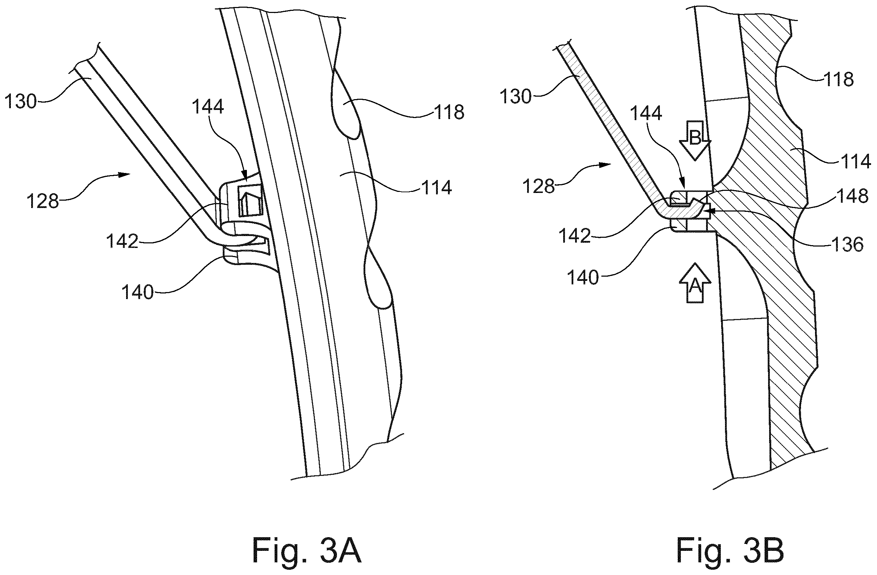

Die in den Figuren 3A und 3B gezeigte zweite Ausführungsform unterscheidet sich von der ersten Ausführungsform dahingehend, dass eine Aussparung 144 in einer Verbindungsplatte 142 so groß ausgebildet ist, dass des freie Ende eines Verbindungsabschnitts 136 eines Federelements 128 nach Umformen des Federelements 128 gänzlich in die Aussparung 144 eintauchen kann. Bei der zweiten Ausführungsform wird durch Umformen also kein Clinchvorsprung 46 sondern eine Abkantung 148 ausgebildet. Die Verhakung bzw. Verrastung des Federelements 128 wird nicht durch Clinchen sondern durch Bördeln bewerkstelligt.

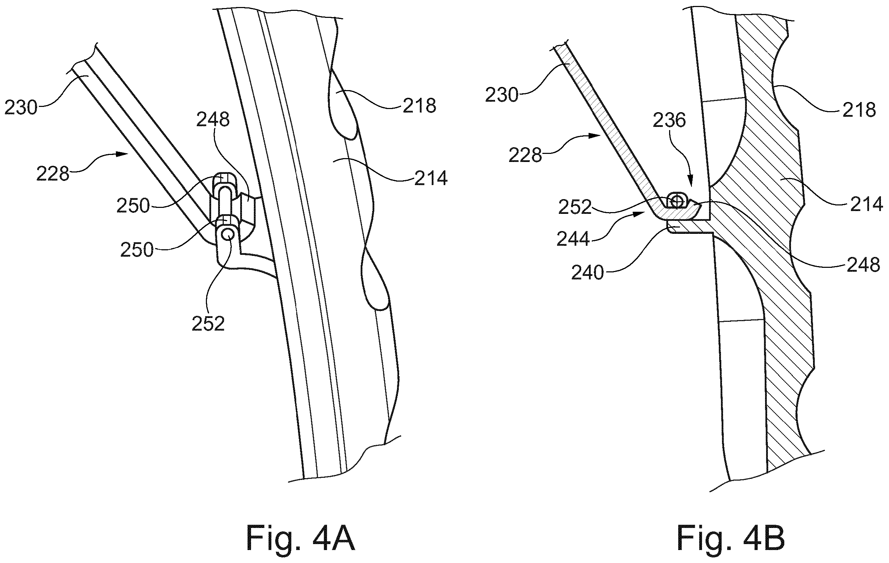

Auch bei der in den Figuren 4A und 4B gezeigten dritten Ausführungsform wird die Verhakung bzw. Verrastung eines Federelements 228 mit einem Griffelement 214 mittels einer gebördelten Abkantung 248 an einem Verbindungsabschnitt 236 bewerkstelligt. Allerdings weist das Griffelement 214 nur eine Verbindungsplatte 240 auf. Die Verbindungsplatte 240 weist an ihrem freien Ende zwei sich im Wesentlichen parallel zum Griffelement 214 erstreckende Vorsprünge 250 auf, welche an ihren freien Enden Durchgangslöcher aufweisen, in die ein Stift 252 derart mit Presspassung eingesteckt werden kann, dass der Stift 252 zusammen mit den Vorsprüngen 250 und der Verbindungsplatte 240 eine Öse 244 zum Verhaken bzw. Verrasten mit der Abkantung 248 bildet. Der Spalt zwischen den Vorsprüngen 250 erstreckt sich parallel zur Erstreckungsrichtung des Griffelements 214 bzw. der Stift 252 erstreckt sich parallel zur Schwenkachse eines Scharniers des Handinstruments gemäß der dritten Ausführungsform (vgl. Scharnier 8 bei der ersten Ausführungsform).

Im Gegensatz zu den ersten beiden Ausführungsformen wird bei der dritten Ausführungsform das Federelement 228 vor dem Montieren mit dem Griffelement 214 umgeformt. Zum Montieren wird das Federelement 228 mit seiner Abkantung 248 zwischen die Vorsprünge 250 auf die Verbindungsplatte 240 bewegt bzw. an die Verbindungsplatte 240 angelegt, so dass ein von der Abkantung 248 eingegrenzter Raum mit Durchgangslöchern an den Enden der Vorsprünge 250 fluchtet. Durch anschließendes Einbringen des Stifts 252 in die Durchgangslöcher wird das abgekantete Ende des Federelements 228 gefangen, wodurch die Verhakung bzw. Verrastung des Federelements 228 mit dem Griffelement 214 zustande kommt.

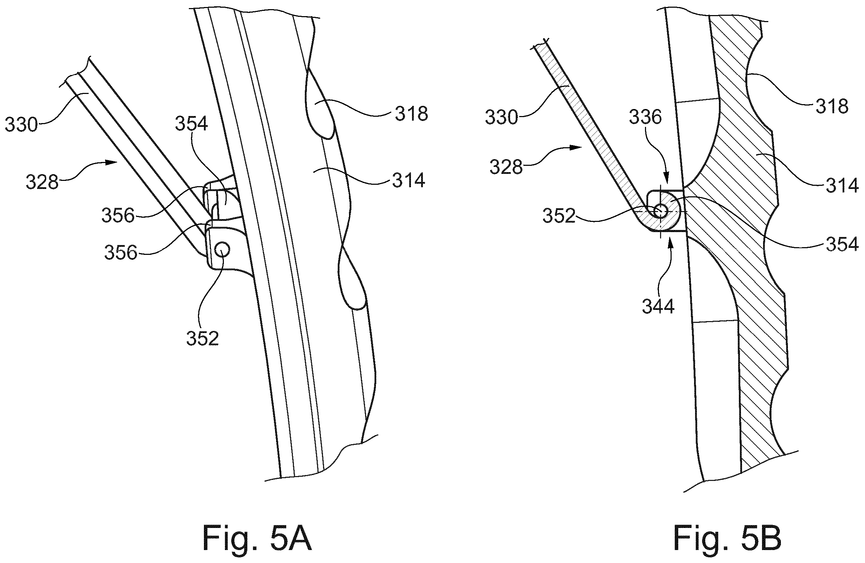

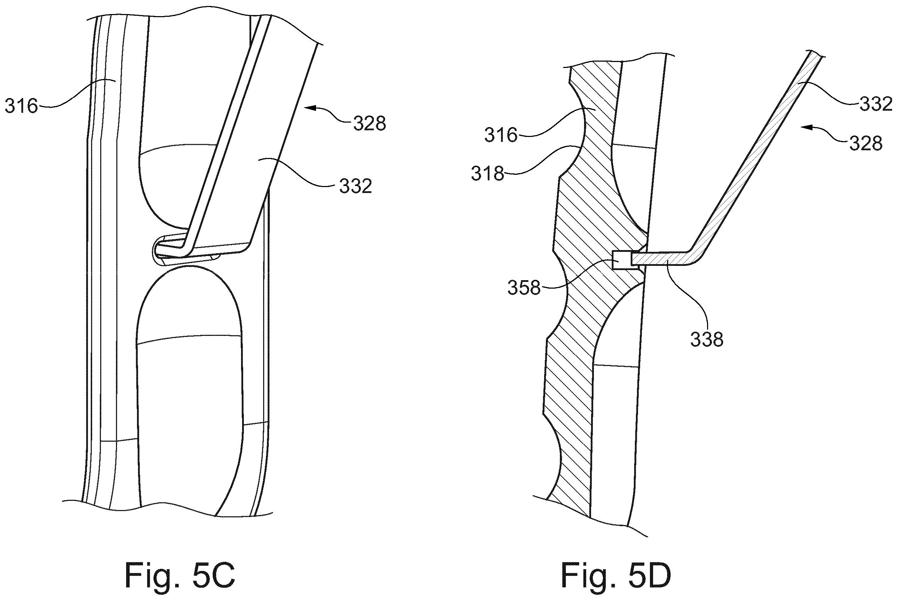

Auch bei der in den Figuren 5A bis 5D gezeigten vierten Ausführungsform der Erfindung wird ein Federelement 328 bereits vor dem Montieren umgeformt. Wie bei der zweiten und der dritten Ausführungsform wird auch bei der vierten Ausführungsform die Verhakung bzw. Verrastung eines Federelements 328 mit einem Griffelement 314 mittels Bördeln bewerkstelligt. Allerdings wird ein freies Ende eines Verbindungsabschnitts 336 nicht nur abgekantet und sondern gänzlich umgeschlagen und weist somit einen Umschlag 354 auf. Das Griffelement 314 weist zwei sich in Richtung eines anderen (in den Figuren 5c und 5D gezeigten) Griffelements 328 erstreckende Vorsprünge 356 auf. Wie bei der dritten Ausführungsform erstreckt sich der Spalt zwischen den Vorsprüngen 356 parallel zur Erstreckungsrichtung des Griffelements 314. Um das Federelement 328 mit dem Griffelement 314 zu verbinden weisen die Vorsprünge 356 an ihren freien Enden Durchgangslöcher auf, in welche ein Stift 352 derart mit Presspassung eingesteckt werden kann, dass der Stift 352 zusammen mit den Vorsprüngen 356 und dem Griffelement 328 eine Öse 344 zum Verhaken bzw. Verrasten mit dem Umschlag 354 bildet. Zum Montieren wird das Federelement 328 mit seinem Umschlag 354 zwischen die Vorsprünge 356 auf das Griffelement 314 bewegt bzw. an das Griffelement angelegt, so dass ein von dem Umschlag 354 eingegrenzter Raum mit den Durchgangslöchern an den Enden der Vorsprünge 356 fluchtet. Durch anschließendes Einbringen des Stifts 352 wird das umgeschlagene Ende des Federelements 328 gefangen, wodurch die Verhakung bzw. Verrastung des Federelements 328 mit dem Griffelement 314 zustande kommt. Da das Griffelement 314 keinerlei Verbindungsplatten aufweist, ist der mit dem Stift 352 verhakte bzw. verrastete Verbindungsabschnitt 336 im montierten Zustand des Federelements 328 um den Stift 352 schwenkbar.

Am Beispiel der vierten Ausführungsform ist gezeigt, dass an einem erfindungsgemäßen Handinstrument lediglich eines von zwei Federelementenden in erfindungsgemäßer Art mit einem Griffelement verhakt bzw. verrastet sein muss. Wie in den Figuren 5C und 5D gezeigt, kann ein Verbindungsabschnitt 338 des Federelements 328 nur so abgekantet sein, dass er in eine an dem Griffelement 316 ausgebildete nachfolgend auch Federelementendenaufnahme genannte Aufnahme 358 lediglich einsteckbar ist und nicht mit dem Griffelement 316 verhakt werden kann.

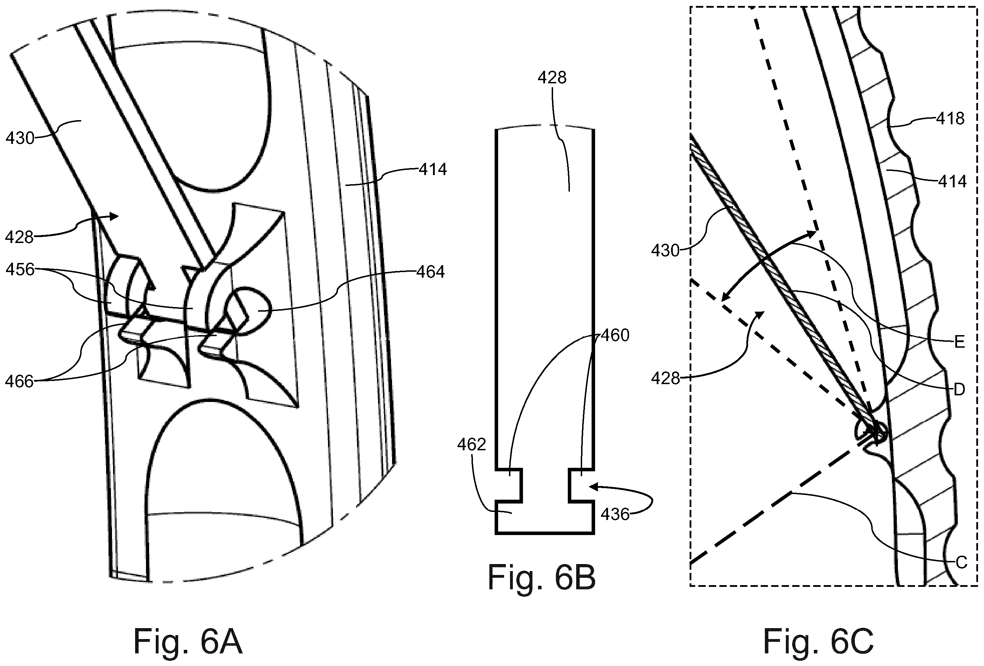

In den Figuren 6A bis 6C ist eine fünfte Ausführungsform gezeigt. Ein Verbindungsabschnitt 436 eines federblattförmigen Federelements 428 weist zwei Ausstanzungen 460 auf, welche derart angeordnet sind, dass ein T-förmiger Endabschnitt 462 am Federelement 428 ausgebildet ist. Anders ausgedrückt weist der Verbindungsabschnitt 436 zwei Vorsprünge auf, welche derart angeordnet sind, dass der T-förmige Endabschnitt 462 gebildet wird. Der Querbalken des T-förmigen Endabschnitts 462 dient dem Federelement 428 im montierten Zustand als Schwenkachse. Das Griffelement 414 weist an der zum anderen (nicht gezeigten) Griffelement weisenden Seite zwei sich in Richtung des anderen Griffelements erstreckende Vorsprünge 456 auf, welche sich parallel zur Erstreckungsrichtung des Griffelements 414 erstrecken. An ihrem jeweiligen freien Ende weisen die Vorsprünge 456 jeweils ein Lagerauge 464 mit jeweils einem sich zum Rand des jeweiligen Vorsprungs 456 erstreckenden Schlitz 466 auf. Der Weite des Schlitzes 466 ist nur unwesentlich größer als die Stärke des Federelements 428.

Zum Verhaken des Federelements 428 gemäß der fünften Ausführungsform mit dem entsprechenden Griffelement 414 wird der Querbalken des T-förmigen Endabschnitts 462 durch den Schlitz 466 in das Lagerauge 464 eingeschoben. Ein Schenkel 430 des Federelements 428 ist bei diesem Einschieben zwangsläufig parallel zum Schlitz 466 auszurichten (siehe angedeutete Montagestellung C in Figur 6C). Gleiches ist bei dem anderen (nicht gezeigten) Griffelement zu tun, um das Federelement 428 zu montieren. Um schließlich das Federelement 428 mit den Griffelementen zu verhaken bzw. zu verrasten, ist das Federelement derart umzuformen, dass sich ihr Schenkel 430 und der (nicht gezeigte) andere Schenkel in Richtung des (nicht gezeigten) Scharniers des Handinstruments gemäß der fünften Ausführungsform erstreckt und der Querbalken des T-förmigen Endabschnitts 462 derart verdreht ist, dass er nicht mehr durch den Schlitz 466 hindurch bewegt werden kann (siehe Grundstellung D in Figur 6C). Der Umstand, dass der Querbalken in den Lageraugen 464 gefangen ist, bleibt solange erhalten, bis die Schenkel des Federelements 428 nicht absichtlich in die Montagestellung C verschwenkt werden. Insbesondere in einem normalen Schwenkbereich E (siehe Figur 6C), in welchem sich die Schenkel des Federelements 428 beim Gebrauchen des Handinstruments gemäß der fünften Ausführungsform bewegen, bleiben die Endabschnitte des Federelements 428 in den Lageraugen der Griffelemente gefangen. Bevorzugter Weise ist das Federelement 428 derart ausgestaltet, dass jede Stellung innerhalb des Schwenkbereichs E stabiler ist als die Montagestellung C. Um trotzdem eine möglich einfache Montage zu ermöglichen, kann das Federelement 428 als Knackfrosch ausgebildet sein, bei welchem die Montagestellung C zumindest eine metastabile Stellung des Federelements 428 darstellt.

Die in den Figuren 1 bis 6C gezeigten und oben beschriebenen Ausführungsformen des medizinischen Handinstruments stellen lediglich fünf mögliche Umsetzungen dar.

### Bezugszeichenliste

- x, xx: Zur ersten Ausführungsform gehörendes Teil
- 2: Handinstrument
- 4, 6: Hebel
- 8: Scharnier
- 10: Maulabschnitt
- 12: Griffabschnitt
- 14,16: Griffelement
- 18: Vertiefung am Griffelement
- 20, 22, 24, 26: Griffvorsprung
- 28: Federelement
- 30, 32: Schenkel des Federelements
- 34: Bogenförmiger Abschnitt des Federelements
- 36, 38: Verbindungsabschnitt des Federelements
- 40, 42: Verbindungsplatte am Griffelement
- 44: Aussparung in der Verbindungsplatte
- 46: Clinchvorsprung
- 1xx: Zur zweiten Ausführungsform gehörendes Teil
- 148: Abkantung
- 2xx: Zur dritten Ausführungsform gehörendes Teil
- 250: Vorsprung an einer Verbindungsplatte
- 252: Stift
- 3xx: Zur vierten Ausführungsform gehörendes Teil
- 354: Umschlag
- 356: Vorsprung an einem Griffelement
- 358: Federelementendenaufnahme
- 4xx: Zur fünften Ausführungsform gehörendes Teil
- 460: Ausstanzung
- 462: T-förmiger Endabschnitt
- 464: Lagerauge
- 466: Schlitz
- A: Einführrichtung eines Werkzeugs zum plastischen Umformen
- B: Einführrichtung eines Werkzeugs zum Zurückformen
- C: Montagestellung
- D: Grundstellung
- E: Federelementschwenkbereich

## Patentansprüche

1. Medizinisches Handinstrument (2) der Zangen- oder Scherenbauart mit
zwei Griffelementen (14, 16; 114; 214; 314; 414), welche relativ zueinander schwenkbar sind,
einem Scharnier (8), welches die Griffelemente (14, 16; 114; 214; 314; 414) schwenkbar verbindet,
Maulteilen bzw. Lastarmen, die bezogen auf das Scharnier gegenüberliegend zu den Griffelementen (414) angeordnet sind, und
einem Federelement (28; 128; 228; 328; 428), welches zwei Federelementenden (36, 38; 136; 436) aufweist, die jeweils mit einem der beiden Griffelemente (14, 16; 114; 214; 314; 414) verbunden sind, so dass bei Verschwenken zumindest eines der beiden Griffelemente (14, 16; 114; 214; 314; 414) aus einer Grundstellung ein Zurückschwenken in die Grundstellung mittels des Federelements (28; 128; 228; 328; 428) bewerkstelligbar ist,
**dadurch gekennzeichnet, dass**
zumindest eines der beiden Federelementenden (36, 38; 136; 236; 336; 436) mit dem entsprechenden Griffelement (14, 16; 114; 214; 314; 414) über einen durch plastisches Umformen des Federelements (28; 128; 228; 328; 428) erzeugten Hinterschnitt formschlüssig verbunden ist und der Hinterschnitt in allen Relativpositionen des Federelements (36, 38; 136; 236; 336; 436) gegenüber dem entsprechenden Griffelement (14, 16; 114; 214; 314; 414) mit diesem Griffelement (14, 16; 114; 214; 314; 414) derart im Eingriff steht, dass eine Demontage ausgeschlossen ist.

2. Medizinisches Handinstrument (2) nach Anspruch 1, **dadurch gekennzeichnet, dass** das Federelement (28; 128; 228; 328; 428) einstückig ausgebildet ist.

3. Medizinisches Handinstrument (2) nach Anspruch 1 oder 2, **dadurch gekennzeichnet, dass** das zumindest eine mit dem entsprechenden Griffelement (14, 16; 114; 214; 314; 414) formschlüssig verbundene Federelementende (36, 38; 136; 236; 336; 436) derart mit dem Griffelement (14, 16; 114; 214; 314; 414) verbunden ist, dass das Federelement (28; 128; 228; 328; 428) bezüglich des Griffelements (14, 16; 114; 214; 314; 414) nur in einer Schwenkebene verschwenkbar ist.

4. Medizinisches Handinstrument (2) nach einem der Ansprüche 1 bis 3,
**dadurch gekennzeichnet, dass** das zumindest eine mit dem entsprechenden Federelementende (36, 38; 136; 236; 336) formschlüssig verbundene Griffelement (14, 16; 114; 214; 314) eine Öse (44; 144; 244; 344) aufweist, mit welcher das Federelementende (36, 38; 136; 236; 336) formschlüssig verbunden ist.

5. Medizinisches Handinstrument (2) nach einem der Ansprüche 1 bis 4,
**dadurch gekennzeichnet, dass** eines der beiden Federelementenden (338) mit dem entsprechenden Griffelement (316) mittels einer Steckverbindung verbunden ist, bei welcher das Federelementende (338) in eine an dem Griffelement (316) ausgebildeten Federelementendenaufnahme (358) hineinragt und durch Verkanten in der Federelementendenaufnahme (358) gehalten ist.

6. Verfahren zum Herstellen eines medizinischen Handinstruments (2) mit den Schritten
- Verbinden zweier Griffelemente (14, 16; 114; 414), so dass diese relativ zueinander schwenkbar sind,
- Ausbilden eines Federelements (28; 128; 428), so dass dieses zwei Federelementenden (36, 38; 136; 436) aufweist,
- plastisches Umformen des Federelements (28; 128; 428), und
- Verbinden eines jeden Federelementendes (36, 38; 136; 436) mit dem entsprechenden Griffelement (14, 16; 114; 414), so dass bei Verschwenken zumindest eines der beiden Griffelemente (14, 16; 114; 414) aus einer Grundstellung ein Zurückschwenken in die Grundstellung mittels des Federelements (28; 128; 428) bewerkstelligbar ist,
wobei das Umformen des Federelements (28; 128; 428) ein formschlüssiges Verbinden zumindest eines der beiden Federelementenden (36, 38; 136; 436) mit dem entsprechenden Griffelement (14, 16; 114; 414) bedingt,
**dadurch gekennzeichnet, dass**
das formschlüssige Verbinden gleichzeitig mit dem plastischen Umformen des Federelements (28; 128; 428) stattfindet.

## Claims

1. A medical hand-held instrument (2) of the forceps or scissor type comprising
two handle elements (14, 16; 114; 214; 314; 414) that can swivel relative to each other,
a hinge (8) which pivotably connects the handle elements (14, 16; 114; 214; 314; 414),
jaw parts or load arms which are arranged opposite to the handle elements (414) with respect to the hinge, and
a spring element (28; 128; 228; 328; 428) having two spring element ends (36, 38; 136; 436) each being connected to one of the two handle elements (14, 16; 114; 214; 314; 414) so that when swiveling at least one of the two handle elements (14, 16; 114; 214; 314; 414) out of an initial position, swiveling back into the initial position can be achieved by means of the spring element (28; 128; 228; 328; 428),
**characterized in that**
at least one of the two spring element ends (36, 38; 136; 236; 336; 436) is positively connected to the corresponding handle element (14, 16; 114; 214; 314; 414) by an undercut created by plastically reshaping the spring element (28; 128; 228; 328; 428) and the undercut is in engagement with said handle element (14, 16; 114; 214; 314; 414) in all positions of the spring element (36, 38; 136; 236; 336; 436) relative to the corresponding handle element (14, 16; 114; 214; 314; 414) such that dismounting is excluded.

2. The medical hand-held instrument (2) according to claim 1, **characterized in that** the spring element (28; 128; 228; 328; 428) is formed in one piece.

3. The medical hand-held instrument (2) according to claim 1 or 2, **characterized in that** the at least one spring element end (36, 38; 136; 236; 336; 436) positively connected to the corresponding handle element (14, 16; 114; 214; 314; 414) is connected to the handle element (14, 16; 114; 214; 314; 414) such that the spring element (28; 128; 228; 328; 428) can be swiveled relative to the handle element (14, 16; 114; 214; 314; 414) in one swivel plane only.

4. The medical hand-held instrument (2) according to any one of the claims 1 to 3, **characterized in that** the at least one handle element (14, 16; 114; 214; 314) positively connected to the corresponding spring element end (36, 38; 136; 236; 336) includes an eyelet (44; 144; 244; 344) to which the spring element end (36, 38; 136; 236; 336) is positively connected.

5. The medical hand-held instrument (2) according to any one of the claims 1 to 4, **characterized in that** one of the two spring element ends (338) is connected to the corresponding handle element (316) by means of a plug connection in which the spring element end (338) projects into a spring element end seat (358) formed at the handle element (316) and is retained by canting within the spring element end seat (358).

6. A method for producing a medical hand-held instrument (2) comprising the steps of
- connecting two handle elements (14, 16; 114; 414) such that they can swivel relative to each other,
- designing a spring element (28; 128; 428) such that it includes two spring element ends (36, 38; 138; 436),
- plastically reshaping the spring element (28; 128; 428), and
- connecting each spring element end (36, 38; 136; 436) to the corresponding handle element (14, 16; 114; 414) such that, when swiveling at least one of the two handle elements (14, 16; 114; 414) out of an initial position, swiveling back into the initial position can be achieved by means of the spring element (28; 128; 428),
wherein reshaping of the spring element (28; 128; 428) requires at least one of the two spring element ends (36, 38;136; 436) to be positively connected to the corresponding handle element (14, 16; 114; 414),
**characterized in that**
the positive connection is carried out simultaneously with the plastic reshaping of the spring element (28; 128; 428).

## Revendications

1. Instrument à main médical (2) du type pince ou ciseaux avec deux éléments de préhension (14, 16 ; 114 ; 214 ; 314 ; 414) qui sont pivotants l'un par rapport à l'autre,
une charnière (8) qui relie de manière pivotante les éléments de préhension (14, 16 ; 114 ; 214 ; 314 ; 414),
des parties de mâchoire ou bras de charge qui sont agencés à l'opposé des éléments de préhension (414) par rapport à la charnière et
un élément de ressort (28 ; 128 ; 228 ; 328 ; 428) qui présente deux extrémités d'élément de ressort (36, 38 ; 136 ; 436) qui sont reliées respectivement à l'un des deux éléments de préhension (14, 16 ; 114 ; 214 ; 314 ; 414) de sorte que lors du pivotement au moins l'un des deux éléments de préhension (14, 16 ; 114 ; 214 ; 314 ; 414) un pivotement retour dans la position de base peut être réalisé au moyen de l'élément de ressort (28 ; 128 ; 228 ; 328 ; 428) à partir d'une position de base,
**caractérisé en ce que**
au moins l'une des deux extrémités d'élément de ressort (36, 38 ; 136 ; 236 ; 336 ; 436) est reliée par complémentarité de formes à l'élément de préhension correspondant (14, 16 ; 114 ; 214 ; 314 ; 414) par le biais d'une contre-dépouille générée par formage plastique de l'élément de ressort (28 ; 128 ; 228 ; 328 ; 428), et la contre-dépouille est en prise dans toutes les positions relatives de l'élément de ressort (36, 38 ; 136 ; 236 ; 336 ; 436) par rapport à l'élément de préhension correspondant (14, 16 ; 114 ; 214 ; 314 ; 414) avec cet élément de préhension (14, 16 ; 114 ; 214 ; 314 ; 414) de telle manière qu'un démontage soit exclus.

2. Instrument à main médical (2) selon la revendication 1, **caractérisé en ce que** l'élément de ressort (28 ; 128 ; 228 ; 328 ; 428) est réalisé d'un seul tenant.

3. Instrument à main médical (2) selon la revendication 1 ou 2, **caractérisé en ce que** la au moins une extrémité d'élément de ressort (36, 38 ; 136 ; 236 ; 336 ; 436) reliée par complémentarité de formes à l'élément de préhension correspondant (14, 16 ; 114 ; 214 ; 314 ; 414) est reliée à l'élément de préhension (14, 16 ; 114 ; 214 ; 314 ; 414) de telle manière que l'élément de ressort (28 ; 128 ; 228 ; 328 ; 428) soit uniquement pivotant dans un plan de pivotement par rapport à l'élément de préhension (14, 16 ; 114 ; 214 ; 314 ; 414).

4. Instrument à main médical (2) selon l'une quelconque des revendications 1 à 3, **caractérisé en ce que** l'au moins un élément de préhension (14, 16 ; 114 ; 214 ; 314) relié par complémentarité de formes à l'extrémité d'élément de ressort correspondante (36, 38 ; 136 ; 236 ; 336) présente un œillet (44 ; 144 ; 244 ; 344) auquel l'extrémité d'élément de ressort (36, 38 ; 136 ; 236 ; 336) est reliée par complémentarité de formes.

5. Instrument à main médical (2) selon l'une quelconque des revendications 1 à 4, **caractérisé en ce qu'**une des deux extrémités d'élément de ressort (338) est reliée à l'élément de préhension (316) correspondant au moyen d'une liaison enfichée, pour laquelle l'extrémité d'élément de ressort (338) pénètre dans un logement d'extrémité d'élément de ressort (358) réalisé au niveau de l'élément de préhension (316) et est maintenue par inclinaison dans le logement d'extrémité d'élément de ressort (358).

6. Procédé de fabrication d'un instrument à main (2) médical avec les étapes suivantes
- liaison de deux éléments de préhension (14, 16 ; 114 ; 414) de sorte que ceux-ci soient pivotants l'un par rapport à l'autre,
- la réalisation d'un élément de ressort (28 ; 128 ; 428) de sorte que celui-ci présente deux extrémités d'élément de ressort (36, 38 ; 136 ; 436),
- le formage plastique de l'élément de ressort (28 ; 128 ; 428), et
- la liaison de chaque extrémité d'élément de ressort (36, 38 ; 136 ; 436) à l'élément de préhension (14, 16 ; 114 ; 414) correspondant de sorte que lors du pivotement au moins l'un des deux éléments de préhension (14, 16 ; 114 ; 414) d'une position de base, un pivotement retour dans la position de base peut être réalisé au moyen de l'élément de ressort (28 ; 128 ; 428),
dans lequel le formage de l'élément de ressort (28 ; 128 ; 428) conditionne une liaison par complémentarité de formes au moins d'une des deux extrémités d'élément de ressort (36, 38 ; 136 ; 436) avec l'élément de préhension (14, 16 ; 114 ; 414) correspondant,
**caractérisé en ce que**
la liaison par complémentarité de formes se produit simultanément au formage plastique de l'élément de ressort (28 ; 128 ; 428).
